# EUROPÄISCHE PATENTSCHRIFT

(11) **EP 0 598 338 B1**
(45) Veröffentlichungstag und Bekanntmachung des Hinweises auf die Patenterteilung: **12.03.1997**
(21) Anmeldenummer: 93118284.4
(22) Anmeldetag: 11.11.1993
(51) Int. Cl.: C07C 17/00, C07C 25/13

(54) **Verfahren zur Herstellung von 1,3-Difluorbenzol**
Process for the preparation of 1,3 - difluorbenzene
Procédé de préparation de 1,3 - difluorbenzène

(30) Priorität: 18.11.1992 DE 4238864
(43) Veröffentlichungstag der Anmeldung: 25.05.1994
(73) Patentinhaber: HOECHST AKTIENGESELLSCHAFT, 65926 Frankfurt am Main (DE)
(72) Erfinder: Schach, Thomas, Dr., D-64579 Gernsheim (DE); Papenfuhs, Theodor, Dr., D-60433 Frankfurt am Main (DE)

(56) Entgegenhaltungen:
- US-A- 2 725 405
- US-A- 5 091 580
- CHEMICAL ABSTRACTS, vol. 115, no. 15, 14. Oktober 1991, Columbus, Ohio, US; abstract no. 158695, NASU R ET AL 'Preparation of m-difluorobenzene' & JP-A-9 177 836 (ISHIHARA SANGYO KAISHA, LTD.;JAPAN ) 3. April 1991

## Beschreibung

Die Erfindung betrifft ein verbessertes Verfahren zur Herstellung von 1,3-Difluorbenzol durch katalytische Abspaltung von Halogen aus halogensubstituierten 1,3-Difluorbenzolen.

1,3-Difluorbenzol stellt ein wichtiges Zwischenprodukt zur Herstellung pharmazeutischer Produkte dar.

Verfahren zur Herstellung von 1,3-Difluorbenzol sind an sich bekannt. Eine der bekannten klassischen Synthesen zur Herstellung von 1,3-Difluorbenzol führt über 2,4-Difluornitrobenzol, das zum entsprechenden Amin reduziert, diazotiert und reduktiv verkocht wird. Die technische Durchführung dieses Verfahrens ist problematisch wegen der Instabilität der Diazoniumsalze und dem Anfall saurer Abwässer, die nur schwer zu entsorgen sind.

1,3-Difluorbenzol kann auch durch direkten Cl-/F-Austausch an 1,3-Dichlorbenzol hergestellt werden. Allerdings benötigt man hierbei sehr drastische Reaktionsbedingungen und es sind nur mäßige Ausbeuten erzielbar (Pews, R. G.; Gall, J. A.; J. Fluorine Chem., 50 (3), 371 - 5; EP 371563).

Ein alternativer Herstellungsweg führt über das 3-Fluoranilin, welches nach Diazotierung in Gegenwart von Fluorwasserstoff durch thermische Zersetzung das gewünschte 1,3-Difluorbenzol liefert (JP 01283230).

In jüngerer Zeit sind Herstellungsmethoden beschrieben worden, die als Vorstufe 2,4-Difluorbenzaldehyd verwenden, dessen katalytische Decarbonylierung bei hohen Temperaturen ebenfalls einen Zugang zum gewünschten 1,3-Difluorbenzol darstellt (DE 3935862; DE 3824141; US 4847442).

Die vorstehend erwähnten, bekannten Verfahren liefern zum Teil nur mäßige Ausbeuten oder verwenden technisch schwierig herzustellende und damit kostspielige Ausgangsverbindungen. So lassen beispielsweise beim Decarbonylierungsverfahren das bislang hohe Preisniveau der Ausgangsverbindung 2,4-Difluorbenzaldehyd, Korrosionsprobleme und die hohen Katalysatorkosten dieses Verfahren für eine technische Realisierung wenig attraktiv erscheinen.

In der JP-OS Hei-3-77836 wird ein Verfahren zur Herstellung von 1,3-Difluorbenzol durch reduktive Enthalogenierung von Chlor-m-difluorbenzolen, wie beispielsweise 2,4-Difluorchlorbenzol, mit Wasserstoffgas in Gegenwart eines Katalysators und einer Base (als Chlorwasserstoff-Binder) beschrieben, wobei als Katalysatoren ein Platin-Trägerkatalysator, ein Nickel-Chrom-Trägerkatalysator oder ein Raney-Nickel-Trägerkatalysator, und als Base (Säurefänger) Alkalimetallhydroxyde oder -carbonate, wie Natrium- oder Kaliumhydroxid, Natrium- oder Kaliumcarbonat, zur Anwendung gelangen. Der praktischen Anwendung dieses Verfahrens sind jedoch enge Grenzen gesetzt, weil einerseits die Umsetzungen nur schwer reproduzierbar sind (siehe Vergleichsbeispiele 1 bis 3) und andererseits neben der Korrosion nur eine mäßige Produktqualität (Fluorbenzolgehalt) erreichbar ist.

In Anbetracht der vorstehend erwähnten Nachteile der bekannten Verfahren bestand ein großes Bedürfnis nach einem besseren Verfahren, bei dem die den bekannten Verfahren innewohnenden Nachteile vermieden werden und wobei neben guten bis sehr guten Ausbeuten und sehr guter Reinheit auch leicht zugängliche und im technischen Maßstab zur Verfügung stehende Vorstufen verwendet werden.

Es wurde nun überraschenderweise gefunden, daß man 1,3-Difluorbenzol in sehr guten Ausbeuten und in sehr hoher Reinheit, ohne merkliche Korrosion der Reaktionsbehälter, durch Abspaltung von Halogen aus einem 1,3-Difluorhalogenbenzol herstellen kann, indem man ein 1,3-Difluorhalogenbenzol der allgemeinen Formel (1) 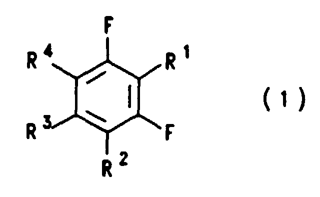 in welcher R¹ bis R⁴ unabhängig voneinander H, Cl oder Br bedeuten und mindestens einer der Reste R¹ bis R⁴ für Cl oder Br steht, in Gegenwart eines Palladium-Katalysators und eines Amins oder eines Gemisches verschiedener Amine, gegebenenfalls in Anwesenheit von Wasser oder eines gegenüber den Reaktionsteilnehmern unter den vorherrschenden Reaktionsbedingungen inerten organischen Lösungsmittels, mit Wasserstoff unter Druck und bei Temperaturen von etwa 70° bis etwa 140°C, vorzugsweise von etwa 90° bis etwa 120°C, besonders bevorzugt von etwa 95° bis 115°C, umsetzt.

Bei den Ausgangsverbindungen kann es sich um Mischungen der verschiedensten chlor- und/oder bromsubstituierten meta-Difluorbenzole handeln. Es können aber auch reine chlor- oder bromsubstituierte meta-Difluorbenzole eingesetzt werden. An Einzelverbindungen der Ausgangsverbindungen der genannten allgemeinen Formel (1) seien beispielsweise genannt ein 1,3-Difluorchlorbenzol, 1,3-Difluordichlorbenzol, 1,3-Difluorbrombenzol, Difluorbrombenzol, wie beispielsweise 2,4-Difluorchlorbenzol, 2,6-Dichlor-1,3-difluorbenzol oder 4,6-Dichlor-1,3-difluorbenzol.

Es ist zweckmäßig, den Katalysator auf einem Trägermaterial, wie beispielsweise Aktivkohle, Calciumcarbonat, Bariumsulfat, Bimsstein, Tonerde, Kieselgur, Kieselgel und/oder Aluminiumoxid anzuwenden. Bevorzugt wird Palladium auf Aktivkohle oder Aluminiumoxid als Trägermaterial zur Anwendung gebracht.

Es ist ferner zweckmäßig, einen Palladium-Trägerkatalysator anzuwenden, der etwa 0,1 bis etwa 10 Gew.-%, vorzugsweise etwa 0,2 bis etwa 8 Gew.-%, besonders bevorzugt etwa 0,5 bis etwa 6 Gew.-% Palladium, bezogen auf den gesamten Katalysator, enthält.

Man setzt etwa 10 bis etwa 10 000 ppm Palladium pro Mol 1,3-Difluorhalogenbenzol und etwa 1 bis etwa 50 000, vorzugsweise etwa 10 bis etwa 10 000 ppm Palladium, bezogen auf Äquivalente abzuspaltendes Halogen, ein.

An Aminen können Monoamine oder Polyamine mit zwei bis etwa vier Aminogruppen oder Gemische daraus dienen.

Besonders geeignet sind Amine der allgemeinen Formel (2)

NHₓR_{y} (2)

in welcher R unabhängig voneinander einen geradkettigen oder verzweigten Alkylrest-CₙH₂ₙ₊₁, worin n für eine Zahl von etwa 6 bis etwa 20, vorzugsweise von etwa 7 bis etwa 16, und besonders bevorzugt von etwa 8 bis etwa 12 steht, x = 0,1 oder 2, y = 1,2 oder 3 und x+y = 3 darstellen.

An hochwirksamen aliphatischen Aminen seien im einzelnen Tri-(N-dodecyl)-amin und Trialkyl(C₈-C₁₀)-amine genannt.

Obwohl die vorstehend genannten Alkylamine der genannten Formel (2) am geeignetsten sind, können prinzipiell auch Arylamine und Aralkylamine eingesetzt werden.

Die Aminkonzentration kann frei gewählt werden. Bevorzugt wird das Amin ohne Zusatz eines Lösungs- oder Verdünnungsmittels verwendet.

Hinsichtlich des Mengenverhältnisses Amin:Ausgangsverbindung ist es zweckmäßig, in Gegenwart von etwa 50 bis etwa 250 mol-% Amin, bezogen auf Äquivalente abzuspaltendes Halogen, umzusetzen.

Gegebenenfalls zur Anwendung gelangende inerte organische Lösungsmittel sind beispielsweise Toluol, Xylole, Ethylbenzol, Alkanole(C₁-C₄), Dialkyl(C₁-C₄)-ether, Tetrahydrofuran oder Polyethylenglykoldimethylether mit 1-15 (C₁-C₄)-Gliedern.

Wie oben ausgeführt kann man auch in Gegenwart von Wasser umsetzen; dabei ist aber das Arbeiten bei einem möglichst geringen Wassergehalt (< 1 %, bezogen auf die Reaktionslösung) bevorzugt.

Das Verfahren kann sowohl bei Atmosphärendruck als auch bei Überdruck vorgenommen werden. Es ist zweckmäßig, bei einem Wasserstoffdruck von etwa 0,1 bis etwa 50 bar umzusetzen.

Das Verfahren kann innerhalb der vorstehend angegebenen allgemeinen und bevorzugten Temperaturbereiche durchgeführt werden. Die Anwendung zu tiefer Temperaturen hat eine langsame und unvollständige Umsetzung zur Folge. Zu hoch gewählte Temperaturen können unerwünschte Fluoridabspaltung bewirken.

Das erfindungsgemäße Verfahren kann in An- oder Abwesenheit von Luftsauerstoff durchgeführt werden. Bevorzugt wird das Arbeiten unter einer Schutzgasatmosphäre, wobei als Schutzgas Stickstoff oder Argon verwendet werden kann.

Als Reduktionsmittel können außer Wasserstoff auch andere Reduktionsmittel, die als Wasserstofflieferanten dienen können, verwendet werden, wie beispielsweise Alkohole, Glykole, Formiate und Hydrazinhydrat.

Zur Regenerierung wird das im Zuge der Reaktion anfallende Aminhydrohalogenid mit einer anorganischen Base, wie beispielsweise Natronlauge oder Magnesiumoxid, behandelt. Aus diesem Grunde ist es zweckmäßig, daß sowohl das freie Amin als auch sein Hydrohalogenid nicht wasserlöslich sind, um so entstehende Aufarbeitungsprobleme zu vermeiden.

Die beim erfindungsgemäßen Verfahren eingesetzten Ausgangsverbindungen der genannten allgemeinen Formel (1) können durch Behandlung eines Nitro-meta-difluorbenzols oder einer Mischung unterschiedlicher Nitro-meta-difluorbenzole bzw. Nitro-meta-difluorhalogenbenzole der allgemeinen Formel (3)

m-(F)₂-Ar-(NO₂)_{z}Rₖ (3)

in welcher R = Cl oder Br, z = 1 oder 2 und k = 0,1 oder 2 bedeuten, mit Chlorgas in Abwesenheit von Lewis-Säuren bei Temperaturen von etwa 80 bis etwa 250°C hergestellt werden.

Zur Herstellung dieser Ausgangsverbindungen sei im einzelnen noch folgendes dargelegt:

Das verwendete Chlorgas wird wasserfrei eingesetzt. Die Umsetzung der m-Difluornitroaromaten mit dem Chlor muß in Abwesenheit von Lewis-Säuren oder anderen Chlorierungskatalysatoren durchgeführt werden. Die benötigten Temperaturen liegen im Bereich 80° bis 250°C, bevorzugt 100° bis 200°C. Die Reaktion kann in Gegenwart oder Abwesenheit eines Fluoridfängers vorgenommen werden. Die benötigte Chlordifluorbenzolverbindung (Ausgangsverbindung) kann beispielsweise wie folgt hergestellt werden:
In einem 2 l Vierhalskolben mit Rührer, Gaseinleitungsrohr und Destillationsbrücke werden 1800 g (11,3 mol) 2,4-Difluornitrobenzol (wasserfrei, frei von Lewissäuren) vorgelegt. Die Reaktionslösung wird auf 160°C gebracht. Dann wird ein Chlorstrom von 4-6 l/h bei dieser Temperatur durch die Lösung geleitet. Nach 1-2 h bilden sich braune Gase, die in verdünnter Natronlauge absorbiert werden. Nach 28 h wird die Gaseinleitung abgebrochen und die Reaktionslösung andestilliert (Kopftemperatur 127°C). Gewonnen werden 675,8 g 2,4-Difluorchlorbenzol, was einer Ausbeute von 91 %, bezogen auf umgesetztes 2,4-Difluornitrobenzol, entspricht.

Die nachstehenden Beispiele dienen zur Erläuterung des erfindungsgemäßen Verfahrens.

### Beispiel 1

Zur Herstellung von 1,3-Difluorbenzol werden 148,5 g (1,0 mol) 2,4-Difluorchlorbenzol, 4,0 g Pd/C (5 %ig, 50 % wasserfeucht) als Katalysator, zusammen mit 626,4 g 1,2 mol Tri-(N-dodecyl)-amin als Base im Reaktionsgefäß (Autoklav) vorgelegt. Die Reaktionslösung wird auf 100°C aufgeheizt und bei dieser Temperatur mit Wasserstoff reduktiv entchloriert. Nach beendeter Wasserstoffaufnahme wird kurz nachgerührt, auf 50 - 60°C gekühlt, die Reaktionslösung mit Natronlauge ausgeschüttelt und der Katalysator vom Reaktionsgemisch abgenutscht. Nach dem Abtrennen der organischen Phase wird diese bei Normaldruck andestilliert, das erhaltene Destillat getrocknet und anschließend fraktioniert. Verbleibende Mutterlauge, Vorläufe, Zwischenläufe und Destillationsrückstände können in Folgeansätzen zurückgeführt werden.
Umsatz: 92,1 % (nach GC)
Ausbeute: 97,1 g (0,85 mol) 1,3-Difluorbenzol
85,2 %, bezogen auf eingesetztes 2,4-Difluorchlorbenzol
Reinheit: 0,2 (GC-Flächen-%) Unbekannte Verbindungen
99,7 (GC-Flächen-%) 1,3-Difluorbenzol
0,1 (GC-flächen-%) Fluorbenzol
Korrosion: Materialuntersuchungen bei 100°C

| | | |
|---|---|---|
| 1.4439 | Abtrag: | < 0,01 mm/a |
| 1.4571 | Abtrag: | < 0,01 mm/a |

### Beispiel 2

Zur Herstellung von 1,3-Difluorbenzol werden 267,3 g (1,8 mol) 2,4-Difluorchlorbenzol, 4,1 g Pd/C (5 %ig, 50 % wasserfeucht) als Katalysator, zusammen mit 871,2 g 2,2 mol einer Mischung von Trialkyl(C₈-C₁₀)aminen als Base im Reaktionsgefäß (Autoklav) vorgelegt. Die Reaktionslösung wird auf 100°C aufgeheizt und bei dieser Temperatur mit Wasserstoff reduktiv entchloriert. Nach beendeter Wasserstoffaufnahme wird kurz nachgerührt, auf Raumtemperatur abgekühlt, die Reaktionslösung mit Natronlauge ausgeschüttelt und der Katalysator vom Reaktionsgemisch abgenutscht. Nach dem Abtrennen der organischen Phase wird diese bei Normaldruck andestilliert, das erhaltene Destillat getrocknet und anschließend fraktioniert. Verbleibende Mutterlauge, Vorläufe, Zwischenläufe und Destillationsrückstände können in Folgeansätzen zurückgeführt werden.
Umsatz: 96,0 % (nach GC)
Ausbeute: 186,7 g (1,64 mol)
91,4 % bezogen auf eingesetztes 2,4-Difluorchlorbenzol
Reinheit: 99,9 (GC-Flächen-%) 1,3-Difluorbenzol
0,05 (GC-Flächen-%) Fluorbenzol
Korrosion: Materialuntersuchungen bei 100°C

| | | |
|---|---|---|
| 1.4439 | Abtrag: | < 0,01 mm/a |
| 1.4529 | Abtrag: | < 0,01 mm/a |

### Vergleichsbeispiel 1

Zur Herstellung von 1,3-Difluorbenzol werden 415 g (2,8 mol) 2,4-Difluorchlorbenzol, 10 g Pd/C (5 %ig, 50 % wasserfeucht) zusammen mit 120 g NaOH in 500 ml Wasser im Reaktionsgefäß (Autoklav) vorgelegt. Die Mischung der Reaktionskomponenten wird in Gegenwart von Wasserstoff langsam auf 100°C erwärmt. Die Reaktionssuspension wird bei dieser Temperatur gehalten bis keine Wasserstoffaufnahme (reduktive Entchlorierung) mehr zu beobachten ist. Anschließend wird der Katalysator vom Reaktionsgemisch abgetrennt und die organische Phase fraktioniert destilliert.
- Umsatz:: 85,3 % (nach GC)
- Ausbeute:: 206,2 g (1,8 mol) 1,3-Difluorbenzol
64,6 % bezogen auf eingesetztes 2,4-Difluorchlorbenzol
- Reinheit:: 0,2 (GC-Flächen-%) Unbekannte Verbindungen
99,2 (GC-Flächen-%) 1,3-Difluorbenzol
0,6 (GC-Flächen-%] Fluorbenzol

### Vergleichsbeispiel 2

Umsetzung analog Vergleichsbeispiel 1. Die Reaktionslösung wurde 1 Stunde bei 100°C gehalten. Es findet keine Wasserstoffaufnahme statt.
- Umsatz:: 4,5 % (nach GC)

### Vergleichsbeispiel 3

Die Reaktionslösung von Vergleichsbeispiel 2 wird mit frischem Katalysator versetzt und nochmals in Gegenwart von Wasserstoff auf 100°C erhitzt. Es findet jedoch kein Umsatz statt. Anschließend wird die Temperatur auf 120°C gesteigert, wobei langsam eine Reaktion eintritt, die jedoch nach etwa 2 Stunden abbricht. Es findet nur geringe Wasserstoffaufnahme statt.
- Umsatz:: 30,2 % (nach GC)

### Vergleichsbeispiel 4

Zur Herstellung von 1,3-Difluorbenzol werden 297,1 g (2,0 mol) 2,4-Difluorchlorbenzol, 6,0 g Pd/C (5 %ig, 50 % wasserfeucht) zusammen mit 212,5 g Ammoniak 25 %ig im Reaktionsgefäß (Autoklav) vorgelegt. Die Mischung der Reaktionskomponenten wird auf 105°C aufgeheizt und bei dieser Temperatur mit Wasserstoff behandelt. Nach beendeter Wasserstoffaufnahme wird der Katalysator vom Reaktionsgemisch abgenutscht, die organische Phase abgetrennt und fraktioniert destilliert.
- Umsatz:: 94,6 % (nach GC)
- Ausbeute:: 182,9 g (1,6 mol) 1,3-Difluorbenzol
80,2 % bezogen auf eingesetztes 2,4-Difluorchlorbenzol
- Reinheit:: 0,3 (GC-Flächen-%) Unbekannte Verbindungen
99,2 (GC-Flächen-%) 1,3-Difluorbenzol
0,5 (GC-Flächen-%) Fluorbenzol

### Vergleichsbeispiel 5

Die Reaktion von Vergleichsbeispiel 4 wird unter gleichen Bedingungen wiederholt. Es findet jedoch bei 105°C keine Wasserstoffaufnahme statt. Erst eine Erhöhung der Reaktionstemperatur auf 140°C ermöglicht die reduktive Enthalogenierung.
- Umsatz:: 68,0 % (nach GC)
- Ausbeute:: 123,1 g (1,1 mol) 1,3-Difluorbenzol
54,0 % bezogen auf eingesetztes 2,4-Difluorchlorbenzol
- Reinheit:: 0,2 (GC-Flächen-%) Unbekannte Verbindungen
99,2 (GC-Flächen-%) 1,3-Difluorbenzol
0,6 (GC-Flächen-%) Fluorbenzol

### Vergleichsbeispiel 6

Zur Herstellung von 1,3-Difluorbenzol werden 594,1 g (4,0 mol) 2,4-Difluorchlorbenzol, 11,9 g Pd/C (5 %ig, 50 % wasserfeucht) zusammen mit 89,0 g 2,2 mol MgO in 450 ml Wasser im Reaktionsgefäß (Autoklav) vorgelegt. Die Mischung der Reaktionskomponenten wird auf 140°C aufgeheizt und bei dieser Temperatur mit Wasserstoff reduktiv entchloriert. Nach beendeter Wasserstoffaufnahme wird der Katalysator vom Reaktionsgemisch abgenutscht, die organische Phase abgetrennt und fraktioniert destilliert.
Umsatz: 96,4 % (nach GC)
Ausbeute: 388,5 g (3,4 mol) 1,3-Difluorbenzol
85,2 % bezogen auf eingesetztes 2,4-Difluorchlorbenzol
Reinheit: 0,2 (GC-Flächen-%) Unbekannte Verbindungen
99,6 (GC-Flächen-%) 1,3-Difluorbenzol
0,2 (GC-Flächen-%) Fluorbenzol
Korrosion: Materialuntersuchungen bei 140°C

| | |
|---|---|
| 1.4439 | Spannungsrißkorrosion |
| 1.4571 | Spannungsrißkorrosion |

### Vergleichsbeispiel 7

Umsetzung erfolgte analog Vergleichsbeispiel 6. Die reduktive Enthalogenierung wurde bei 120°C durchgeführt. (Bei 100°C erfolgt keine Reaktion.)
Umsatz: 91,4 % (nach GC)
Ausbeute: 369,4 g (3,2 mol)
81,0 % bezogen auf eingesetztes 2,4-Difluorchlorbenzol
Reinheit: 0,2 (GC-Flächen-%) Unbekannte Verbindungen
99,6 (GC-Flächen-%) 1,3-Difluorbenzol
0,2 (GC-Flächen-%) Fluorbenzol
Korrosion: Materialuntersuchungen bei 120°C

| | | |
|---|---|---|
| 1.4439 | Abtrag: | 2,0 mm/a |
| 1.4529 | Abtrag: | 1,6 mm/a |
| 2.4856 | Abtrag: | 0,6 mm/a |
| 2.4858 | Abtrag: | 1,2 mm/a |

### Vergleichsbeispiel 8

Zur Herstellung von 1,3-Difluorbenzol werden 572,0 g (3,85 mol) 2,4-Difluorchlobenzol, 11,9 g Pd/C (5 %ig, 50 % wasserfeucht) zusammen mit 95,8 g (4,0 mol) LiOH in 400 ml Wasser im Reaktionsgefäß (Autoklav) vorgelegt. Die Mischung der Reaktionskomponenten wird auf 100°C aufgeheizt und bei dieser Temperatur mit Wasserstoff reduktiv entchloriert. Nach beendeter Wasserstoffaufnahme wird der Katalysator vom Reaktionsgemisch abgenutscht, die organische Phase abgetrennt und fraktioniert destilliert.
- Umsatz:: 79,9 % (nach GC)
- Ausbeute:: 308,2 g (2,7 mol) 1,3-Difluorbenzol
70,2 % bezogen auf eingesetztes 2,4-Difluorchlorbenzol
- Reinheit:: 0,2 (GC-Flächen-%) Unbekannte Verbindungen
99,6 (GC-Flächen-%) 1,3-Difluorbenzol
0,2 (GC-Flächen-%) Fluorbenzol

## Patentansprüche

1. Verfahren zur Herstellung von 1,3-Difluorbenzol hoher Reinheit durch katalytische Abspaltung von Halogen aus einem 1,3-Difluorhalogenbenzol, dadurch gekennzeichnet, daß man ein 1,3-Difluorhalogenbenzol der allgemeinen Formel (1) 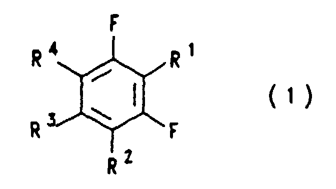 worin R¹ bis R⁴ unabhängig voneinander H, Cl oder Br bedeuten und mindestens einer der Reste R¹ bis R⁴ für Cl oder Br steht, oder Mischungen von 1,3-Difluorhalogenbenzolen der genannten allgemeinen Formel in Gegenwart eines Palladium-Katalysators und eines Amins oder eines Gemisches verschiedener Amine, gegebenenfalls in Anwesenheit von Wasser oder eines gegenüber den Reaktionsteilnehmern und den Reaktionsbedingungen inerten organischen Lösungsmittels, mit Wasserstoff unter Druck und bei Temperaturen von 70° bis 140°C umsetzt.

2. Verfahren nach Anspruch 1, dadurch gekennzeichnet, daß man Mischungen von 1,3-Difluorhalogenbenzolen der in Anspruch 1 genannten allgemeinen Formel (1) umsetzt.

3. Verfahren nach mindestens einem der Ansprüche 1 und 2, dadurch gekennzeichnet, daß man ein 1,3-Difluorchlorbenzol, ein 1,3-Difluordichlorbenzol, ein 1,3-Difluorbrombenzol und/oder ein 1,3-Difluordibrombenzol umsetzt.

4. Verfahren nach mindestens einem der Ansprüche 1 bis 3, dadurch gekennzeichnet, daß man 2,4-Difluorchlorbenzol, 2,6-Dichlor-1,3-difluorbenzol oder 4,6-Dichlor-1,3-difluorbenzol umsetzt.

5. Verfahren nach mindestens einem der Ansprüche 1 bis 4, dadurch gekennzeichnet, daß man bei Temperaturen von 90° bis 120°C umsetzt.

6. Verfahren nach mindestens einem der Ansprüche 1 bis 5, dadurch gekennzeichnet, daß man bei Temperaturen von 95° bis 115°C umsetzt.

7. Verfahren nach mindestens einem der Ansprüche 1 bis 6, dadurch gekennzeichnet, daß man als Palladium-Katalysator einen Palladium-Trägerkatalysator einsetzt.

8. Verfahren nach mindestens einem der Ansprüche 1 bis 7, dadurch gekennzeichnet, daß das Trägermaterial des Palladium-Katalysators aus Bimsstein, Tonerde, Calciumcarbonat, Bariumsulfat, Kieselgur, Kieselgel, Aluminiumoxid und/oder Aktivkohle besteht.

9. Verfahren nach mindestens einem der Ansprüche 1 bis 8, dadurch gekennzeichnet, daß das Trägermaterial des Palladium-Katalysators aus Aluminiumoxid und/oder Aktivkohle besteht.

10. Verfahren nach mindestens einem der Ansprüche 1 bis 9, dadurch gekennzeichnet, daß der Palladium-Katalysator 0,1 bis 10 Gew.-% Palladium, bezogen auf den gesamten Katalysator, enthält.

11. Verfahren nach mindestens einem der Ansprüche 1 bis 10, dadurch gekennzeichnet, daß der Palladium-Katalysator 0,2 bis 8 Gew.-% Palladium, bezogen auf den gesamten Katalysator, enthält.

12. Verfahren nach mindestens einem der Ansprüche 1 bis 11, dadurch gekennzeichnet, daß der Palladium-Katalysator 0,5 bis 6 Gew.-% Palladium, bezogen auf den gesamten Katalysator, enthält.

13. Verfahren nach mindestens einem der Ansprüche 1 bis 12, dadurch gekennzeichnet, daß man 1 bis 50 000 ppm Palladium, bezogen auf Äquivalente abzuspaltendes Halogen, anwendet.

14. Verfahren nach mindestens einem der Ansprüche 1 bis 13, dadurch gekennzeichnet, daß man 10 bis 10 000 ppm Palladium pro mol 1,3-Difluorhalogenbenzol einsetzt.

15. Verfahren nach mindestens einem der Ansprüche 1 bis 14, dadurch gekennzeichnet, daß man als Amin ein Monoamin oder ein Polyamin mit 2 bis etwa 4 Aminogruppen oder Gemische daraus einsetzt.

16. Verfahren nach mindestens einem der Ansprüche 1 bis 15, dadurch gekennzeichnet, daß man in Gegenwart eines Amins der allgemeinen Formel
NHₓR_{y}
in welcher R unabhängig voneinander einen geradkettigen oder verzweigten Alkylrest-CₙH₂ₙ₊₁, worin n für eine Zahl von 6 bis 20 steht, x = 0,1 oder 2, y = 1,2 oder 3 und x+y = 3 darstellen, umsetzt.

17. Verfahren nach Anspruch 16, dadurch gekennzeichnet, daß n in dem dort genannten Alkylrest für eine Zahl von 7 bis 16 steht.

18. Verfahren nach Anspruch 16, dadurch gekennzeichnet, daß n in dem dort genannten Alkylrest für eine Zahl von 8 bis 12 steht.

19. Verfahren nach mindestens einem der Ansprüche 1 bis 18, dadurch gekennzeichnet, daß man in Gegenwart von Tri-(N-dodecyl)-amin umsetzt.

20. Verfahren nach mindestens einem der Ansprüche 1 bis 19, dadurch gekennzeichnet, daß man in Gegenwart von Trialkyl(C₈-C₁₀)-aminen umsetzt.

21. Verfahren nach mindestens einem der Ansprüche 1 bis 20, dadurch gekennzeichnet, daß man in Gegenwart von 50 bis 250 mol-% Amin, bezogen auf Äquivalente abzuspaltendes Halogen, umsetzt.

22. Verfahren nach mindestens einem der Ansprüche 1 bis 21, dadurch gekennzeichnet, daß man in Gegenwart von Toluol, Xylolen, Ethylbenzol, Alkanolen(C₁-C₄), Dialkyl(C₁-C₄)-ethern, Tetrahydrofuran oder Polyethylenglykoldimethylethern mit 1-15 (C₁-C₄)-Gliedern als inertem organischen Lösungsmittel umsetzt.

23. Verfahren nach mindestens einem der Ansprüche 1 bis 22, dadurch gekennzeichnet, daß man bei Atmosphärendruck umsetzt.

24. Verfahren nach mindestens einem der Ansprüche 1 bis 22, dadurch gekennzeichnet, daß man bei Überdruck umsetzt.

25. Verfahren nach mindestens einem der Ansprüche 1 bis 24, dadurch gekennzeichnet, daß man bei einem Wasserstoffdruck von 0,1 bis 50 bar umsetzt.

## Claims

1. A process for preparing 1,3-difluorobenzene of high purity by means of catalytic elimination of halogen from a 1,3-difluorohalobenzene, which comprises reacting a 1,3-difluorohalobenzene of the formula (1) 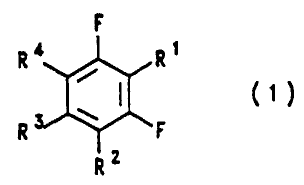 in which R¹ to R⁴, independently of each other, are H, Cl or Br and at least one of the radicals R¹ to R⁴ is Cl or Br, or mixtures of 1,3-difluorohalobenzenes of said formula, in the presence of a palladium catalyst and of an amine or of a mixture of different amines, optionally in the presence of water or of an organic solvent which is inert toward the reactants and the reaction conditions, with hydrogen under pressure and at temperatures from 70° to 140°C.

2. The process as claimed in claim 1, wherein mixtures of 1,3-difluorohalobenzenes of the formula (1) mentioned in claim 1 are reacted.

3. The process as claimed in at least one of claims 1 and 2, wherein a 1,3-difluorochlorobenzene, a 1,3-difluorodichlorobenzene, a 1,3-difluorobromobenzene and/or a 1,3-difluorodibromobenzene is/are reacted.

4. The process as claimed in at least one of claims 1 to 3, wherein 2,4-difluorochlorobenzene, 2,6-dichloro-1,3-difluorobenzene or 4,6-dichloro-1,3-difluorobenzene is reacted.

5. The process as claimed in at least one of claims 1 to 4, wherein the reaction is carried out at temperatures from 90° to 120°C.

6. The process as claimed in at least one of claims 1 to 5, wherein the reaction is carried out at temperatures from 95° to 115°C.

7. The process as claimed in at least one of claims 1 to 6, wherein a palladium-support catalyst is employed as the palladium catalyst.

8. The process as claimed in at least one of claims 1 to 7, wherein the support material of the palladium catalyst comprises pumice stone, alumina, calcium carbonate, barium sulfate, diatomaceous earth, silica gel, aluminum oxide and/or active charcoal.

9. The process as claimed in at least one of claims 1 to 8, wherein the support material of the palladium catalyst comprises aluminum oxide and/or active charcoal.

10. The process as claimed in at least one of claims 1 to 9, wherein the palladium catalyst contains 0.1 to 10% by weight of palladium, based on the whole catalyst.

11. The process as claimed in at least one of claims 1 to 10, wherein the palladium catalyst contains 0.2 to 8% by weight of palladium, based on the whole catalyst.

12. The process as claimed in at least one of claims 1 to 11, wherein the palladium catalyst contains 0.5 to 6% by weight of palladium, based on the whole catalyst.

13. The process as claimed in at least one of claims 1 to 12, wherein 1 to 50,000 ppm of palladium, based on equivalents of halogen to be eliminated, are employed.

14. The process as claimed in at least one of claims 1 to 13, wherein 10 to 10,000 ppm of palladium are employed per mole of 1,3-difluorohalo-benzene.

15. The process as claimed in at least one of claims 1 to 14, wherein a monoamine or a polyamine having 2 to about 4 amino groups, or mixtures thereof, is/are employed as the amine.

16. The process as claimed in at least one of claims 1 to 15, wherein the reaction is carried out in the presence of an amine of the formula
NHₓR_{y}
in which the R radicals, independently of each other, are a straight-chain or branched alkyl radical -CₙH₂ₙ₊₁, in which n is a number from 6 to 20, x = 0,1 or 2, y = 1,2 or 3 and x + y = 3.

17. The process as claimed in claim 16, wherein n in the alkyl radical mentioned in that claim is a number from 7 to 16.

18. The process as claimed in claim 16, wherein n in the alkyl radical mentioned in that claim is a number from 8 to 12.

19. The process as claimed in at least one of claims 1 to 18, wherein the reaction is carried out in the presence of tri-(N-dodecyl)amine.

20. The process as claimed in at least one of claims 1 to 19, wherein the reaction is carried out in the presence of trialkyl(C₈-C₁₀)amines.

21. The process as claimed in at least one of claims 1 to 20, wherein the reaction is carried out in the presence of 50 to 250 mol% of amine, based on equivalents of halogen to be eliminated.

22. The process as claimed in at least one of claims 1 to 21, wherein the reaction is carried out in the presence of toluene, xylenes, ethylbenzene, alkanols(C₁-C₄), dialkyl(C₁-C₄) ethers, tetrahydrofuran or polyethylene glycol dimethyl ethers having 1-15 (C₁-C₄) members as the inert organic solvent.

23. The process as claimed in at least one of claims 1 to 22, wherein the reaction is carried out under atmospheric pressure.

24. The process as claimed in at least one of claims 1 to 22, wherein the reaction is carried out under excess pressure.

25. The process as claimed in at least one of claims 1 to 24, wherein the reaction is carried out at a hydrogen pressure of 0.1 to 50 bar.

## Revendications

1. Procédé de préparation de 1,3-difluorobenzène de haute pureté par clivage catalytique d'halogène à partir d'un 1,3-difluoro-halogénobenzène, caractérisé en ce qu'on fait réagir un 1,3-difluoro-halogénobenzène de formule générale (1) 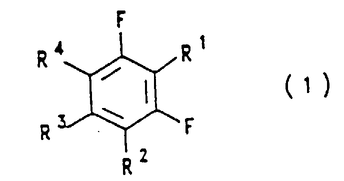 dans laquelle R¹ à R⁴ représentent, indépendamment les uns des autres, H, Cl ou Br et au moins l'un des radicaux R¹ à R⁴ représente Cl ou Br, ou des mélanges de 1,3-difluorohalogénobenzènes de la susdite formule générale, en présence d'un catalyseur au palladium et d'une amine ou d'un mélange d'amines différentes, éventuellement en présence d'eau ou d'un solvant organique inerte vis-à-vis des participants à la réaction et des conditions de réaction, avec l'hydrogène sous pression et à des températures de 70° à 140°C.

2. Procédé selon la revendication 1, caractérisé en ce qu'on fait réagir des mélanges de 1,3-difluorohalogénobenzènes de formule générale (1) mentionnée à la revendication 1.

3. Procédé selon au moins une des revendications 1 et 2, caractérisé en ce qu'on fait réagir un 1,3-difluorochlorobenzène, un 1,3-difluoro-dichlorobenzène, un 1,3-difluoro-bromobenzène et/ou un 1,3-difluoro-dibromobenzène.

4. Procédé selon au moins une des revendications 1 à 3, caractérisé en ce qu'on fait réagir le 2,4-difluorochlorobenzène, le 2,6-dichloro-1,3-difluorobenzène ou le 4,6-dichloro-1,3-difluorobenzène.

5. Procédé selon au moins une des revendications 1 à 4, caractérisé en ce qu'on effectue la réaction à des températures de 90° à 120°C.

6. Procédé selon au moins une des revendications 1 à 5, caractérisé en ce qu'on effectue la réaction à des températures de 95° à 115°C.

7. Procédé selon au moins une des revendications 1 à 6, caractérisé en ce qu'on utilise un catalyseur palladium-support comme catalyseur au palladium.

8. Procédé selon au moins une des revendications 1 à 7, caractérisé en ce que le matériau de support du catalyseur au palladium est constitué de pierre ponce, d'alumine, de carbonate de calcium, de sulfate de baryum, de terre d'infusoires, de gel de silice, d'oxyde d'aluminium et/ou de charbon actif.

9. Procédé selon au moins une des revendications 1 à 8, caractérisé en ce que le matériau de support du catalyseur au palladium est constitué d'oxyde d'aluminium et/ou de charbon actif.

10. Procédé selon au moins une des revendications 1 à 9, caractérisé en ce que le catalyseur au palladium contient 0,1 à 10 % en poids de palladium, par rapport à l'ensemble du catalyseur.

11. Procédé selon au moins une des revendications 1 à 10, caractérisé en ce que le catalyseur au palladium contient 0,2 à 8 % en poids de palladium, par rapport à l'ensemble du catalyseur.

12. Procédé selon au moins une des revendications 1 à 11, caractérisé en ce que le catalyseur au palladium contient 0,5 à 6 % en poids de palladium, par rapport à l'ensemble du catalyseur.

13. Procédé selon au moins une des revendications 1 à 12, caractérisé en ce qu'on utilise 1 à 50 000 ppm de palladium, par rapport aux équivalents d'halogène à cliver.

14. Procédé selon au moins une des revendications 1 à 13, caractérisé en ce qu'on met en oeuvre 10 à 10 000 ppm de palladium par mol de 1,3-difluoro-halogénobenzène.

15. Procédé selon au moins une des revendications 1 à 14, caractérisé en ce qu'on met en oeuvre, en tant qu'amine, une monoamine ou une polyamine avec 2 à environ 4 groupes amino ou des mélanges de celles-ci.

16. Procédé selon au moins une des revendications 1 à 15, caractérisé en ce qu'on effectue la réaction en présence d'une amine de formule générale
N Hₓ R_{y}
dans laquelle les R représentent, indépendamment les uns des autres, un radical alkyle -CₙH₂ₙ₊₁, rectiligne ou ramifié, dans lequel n représente un nombre de 6 à 20, x = 0, 1 ou 2, y = 1, 2 ou 3 et x + y = 3.

17. Procédé selon la revendication 16, caractérisé en ce que n, dans le radical alkyle qui y est mentionné, représente un nombre de 7 à 16.

18. Procédé selon la revendication 16, caractérisé en ce que n, dans le radical alkyle qui y est mentionné, représente un nombre de 8 à 12.

19. Procédé selon au moins une des revendications 1 à 18, caractérisé en ce qu'on effectue la réaction en présence de tri-(N-dodécyl)-amine.

20. Procédé selon au moins une des revendications 1 à 19, caractérisé en ce qu'on effectue la réaction en présence de trialkyl(en C₈-C₁₀)-amines.

21. Procédé selon au moins une des revendications 1 à 20, caractérisé en ce qu'on effectue la réaction en présence de 50 à 250 mol % d'amine, par rapport aux équivalents d'halogène à cliver.

22. Procédé selon au moins une des revendications 1 à 21, caractérisé en ce qu'on effectue la réaction en présence de toluène, de xylènes, d'éthylbenzène, d'alcanols en C₁-C₄, d'éthers de di(alkyles en C₁-C₄), de tétrahydrofurane ou d'éther diméthyliques de polyéthylèneglycol avec 1 à 15 membres en C₁-C₄ comme solvant organique inerte.

23. Procédé selon au moins une des revendications 1 à 22, caractérisé en ce qu'on effectue la réaction à la pression atmosphérique.

24. Procédé selon au moins une des revendications 1 à 22, caractérisé en ce qu'on effectue la réaction en surpression.

25. Procédé selon au moins une des revendications 1 à 24, caractérisé en ce qu'on effectue la réaction sous une pression d'hydrogène de 0,1 à 50 bars.
